# EUROPEAN PATENT APPLICATION

(11) **EP 0 533 920 A1**
(43) Date of publication of application: **31.03.1993**
(21) Application number: 90904673.2
(22) Date of filing: 14.03.1990
(51) Int. Cl.: C07D 495/04, A61K 31/415

(54) **THIOPYRANO 2,3-b]INDOLE DERIVATIVE**

(71) Applicant: YAMANOUCHI PHARMACEUTICAL CO. LTD., Tokyo 103 (JP)
(72) Inventor: OHTA, Mitsuaki, Tsukuba-gun, Ibaraki 300-24 (JP); YANAGISAWA, Isao, Tokyo 177 (JP); MIYATA, Keiji, Tokyo 161 (JP); KOIDE, Tokuo, Tsukuba-shi, Ibaraki 305 (JP); SUZUKI, Takeshi, Tsukuba-shi, Ibaraki 305 (JP); MATSUHISA, Akira, Tsukuba-shi, Ibaraki 305 (JP)
(74) Representative: Geering, Keith Edwin
(86) International application number: JP9000329
(87) International publication number: WO9113888

(57) **Abstract**

This invention relates to a thiopyrano[2,3-b] indole derivative represented by general formula (I) (wherein R¹ and R² are the same or different from each other and each represents a hydrogen atom or a lower alkyl group and two broken lines represent each a single bond or one of them represents a double bond), and pharmacologically acceptable salts thereof. These compounds have an excellent 5-HT₃ antagonism.

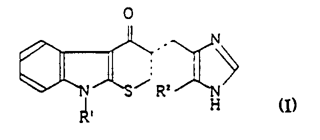

## Description

### (Technical Field)

The present invention relates to novel thiopyrano[2,3-b]indole derivatives and salts thereof, to processes for producing the same, and to 5-HT₃ antagonists containing these compounds.

### (Background Art)

As 5-HT₃ antagonists, are known hitherto azabicyclo compounds described in JP-A-59-36675 and JP-A-59-67284, tetrahydrocarbazole derivatives described in JP-A-60-214784 and JP-A-63-211279, and azabicyclo compounds described in JP-A-61-275276 (the term "JP-A" as used herein means an "unexamined published Japanese patent application").

### (Disclosure of the Invention)

To make a search for compounds having an excellent 5-HT₃ antagonizing action, the present inventors synthesized a variety of novel compounds and subjected them to a screening test, and found that thiopyrano[2,3-b]indole derivatives represented by the following general formula (I) and salts thereof have an excellent 5-HT₃ antagonistic activity. The present invention was accomplished on the basis of these findings. 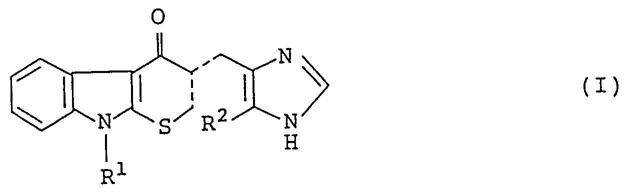
(wherein R¹ and R² are the same or different from each other and each represents a hydrogen atom or a lower alkyl group; and the broken lines mean that both are single bonds or either one of them is a double bond.)

Thus, an object of the present invention is to provide compounds represented by the above general formula (I) and salts thereof, and a further object is to provide pharmaceutical compositions of 5-HT₃ antagonist comprising a compound represented by the above general formula (I) or a salt thereof.

The compounds (I) of the present invention are different from conventional 5-HT₃ antagonizing substances in fundamental structure and are characterized by an excellent 5-HT₃ antagonizing action.

Described below are the compounds and pharmaceuticalS of the present invention in more detail.

The term "lower" as used in the definition of the groups in general formulas in this specification means, unless specifically indicated, a linear or branched alkyl group with a carbon number of 1 to 6.

Accordingly, as examples of "a lower alkyl group", may be mentioned, among others, methyl, ethyl, propyl, butyl, pentyl (amyl), isopropyl, isobutyl, tert-butyl, isopentyl, neo-pentyl, tert-pentyl and hexyl groups.

The compounds (I) of the present invention form their salts, and pharmacologically acceptable salts of compounds (I) are included in the present invention. As examples of such salts, may be mentioned acid addition salts with a mineral acid (such as hydrochloric, hydrobromic, hydroiodic, sulfuric, nitric and phosphoric acids), with an organic acid (such as formic, acetic, propionic, oxalic, malonic, succinic, fumaric, maleic, malic, tartaric, methanesulfonic and ethanesulfonic acids), and with an acidic amino acid (such as aspartic and glutamic acids).

In addition, the compounds of the present invention involve various isomers, such as optical isomers due to the asymmetric carbon atom and keto-enol tautomeric isomers due to the oxo group. The present invention also includes such isomers (both isolated isomers and mixtures thereof).

The compounds of the present invention may be prepared by various methods, and typical preparation methods are described below.

### (Preparation Method 1)

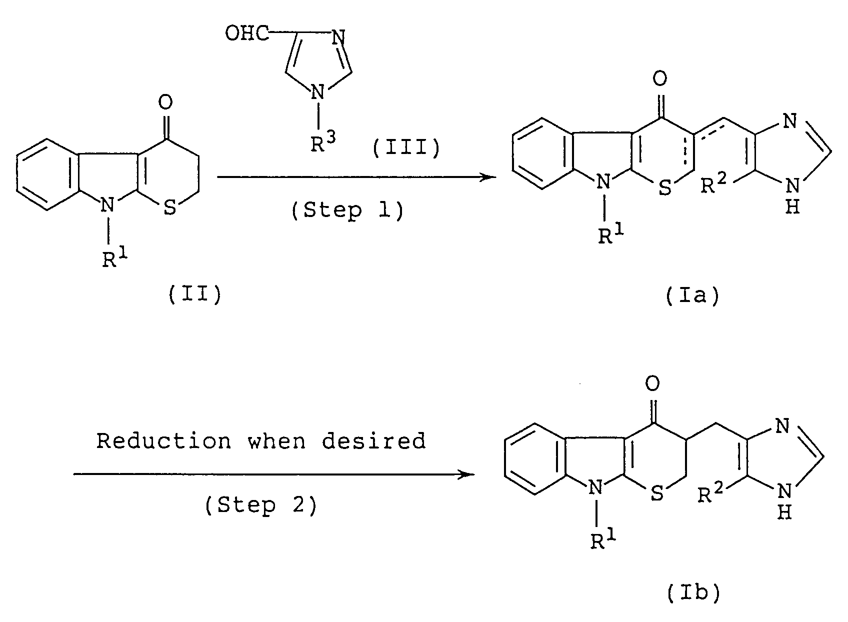

(wherein R¹ and R² are the same as above; R³ is an arylmethyl group; and ----- means that either one of them is a double bond. The same applies hereinafter.)

The compounds (I) of the present invention can be prepared by reacting the tetrahydrothiopyrano[2,3-b]indole compound represented by the general formula (II) with the imidazole derivative represented by the general formula (III) (Step 1), and then reducing the compound (Ia) thus obtained, when desired (Step 2).

### (1) Step 1

The reaction of the compound (II) with the compound (III) is preferably carried out in a solvent inert to the reaction (such as diethyl ether, dioxane, tetrahydrofuran, benzene, toluene, xylenes, chloroform, dichloromethane and dimethylformamide) preferably in the presence of a base. As examples of the base, may be mentioned alkyl lithiums (such as butyl lithium), alkali metal hydrides (such as sodium hydride), alkali metal amides (such as sodium amide and lithium diisopropylamide), alkali metal carbonates (such as sodium carbonate) and alkali metal alkoxides (such as sodium methoxide and potassium methoxide).

The reaction is carried out under cooling or at room temperature. As examples of the arylmethyl group in the compound (III) represented by R³, may be mentioned benzyl, benzhydryl and trityl groups. These groups can be eliminated by treatment with acetic, trifluoroacetic, p-toluenesulfonic or hydrochloric acid, etc., or by reduction according to the conventional method.

### (2) Step 2

The compound represented by the general formula (Ib) can be obtained, when desired, by reducing the compound (Ia) obtained by Step 1. The reduction may be performed by hydrogenation in the presence of a metal catalyst commonly employed (such as palladium, Raney nickel, platinum and rhodium). As the reaction solvent, may be used an alcohol (such as methanol and ethanol), dioxane, diethyl ether, tetrahydrofuran or ethyl acetate, and the reaction is carried out under cooling or heating.

### (Preparation Method 2)

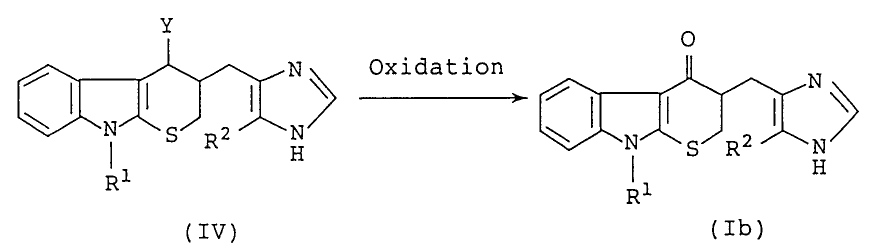

(wherein Y is a hydrogen atom or a hydroxyl group. The same applies hereinafter.)

The compound represented by the general formula (Ib) can be prepared by oxidizing the compound represented by the general formula (IV) in the presence of a suitable oxidizing agent.

The oxidation may be performed by the use of selenium dioxide, ammonium ceric sulfate, an acetone solution of chromic acid or a pyridine solution of chromium trioxide when Y in the compound represented by the general formula (IV) is a hydrogen atom, and by the use of other oxidizing agents, such as N-bromosuccinimide, N-chlorosuccinimide, pyridine-sulfur trioxide complex and copper chromite when Y is a hydroxyl group.

As the reaction solvent, may be used acetone, tetrahydrofuran, dioxane, dimethylformamide, methanol, ethanol or dichloromethane, and the reaction is carried out under cooling or heating.

The compound of the present invention thus formed is isolated and purified in its free form or in the form of a salt thereof. The isolation and purification may be performed by ordinary chemical operations, such as extraction, crystallization, recrystallization and various types of chromatography.

A racemic compound can be converted into stereochemically pure isomers by the use of a proper raw material or by the commonly employed racemic resolution method [for example, a process of leading to a diastereomer with an optically active acid (such as a tartaric acid), followed by optical resolution].

### (Industrial Applicability)

The compounds of the present invention and salts thereof were found to specifically depress the temporary bradycardia in an anesthetized rat caused by serotonin, and are therefore considered to have the 5-HT₃ antagonizing action. Accordingly, it is considered that the compounds of the present invention work to depress the vomit caused by a carcinostatic agent, such as cisplatin, and radioactive rays, and are useful for the prevention and treatment of megrim, compound headache, trigeminal neuralgia, anxiety neurosis, gastrointestinal disorders, peptic ulcers, irritable bowel syndrome, and other diseases.

The pharmacological effects of the compounds of the present invention were confirmed as described below.

### 1) Antagonism against 5-HT₃-receptor

A nine-week-old male Wister rat was anesthetized by intraperitoneal administration of 1 g/kg urethane, and its blood pressure and heart rate were measured while practicing artificial respiration. The temporary decline of the heart rate and blood pressure caused by intravenous administration of serotonin, or 2-methylserotonin which is the selective agonist for 5-HT₃, was judged as an index to the reaction with the 5-HT₃ receptor (Bezold-Jarish reflex; Paintal, A.S., Pysiol. Rev., 53, 159 (1973)].

The compound of the present invention or a salt thereof, when intravenously administered (0.03 to 10 µg/kg) or orally administered (1 to 300 µg/kg) 10 minutes or 60 minutes before the administration of serotonin (or 2-methylserotonin), respectively, depressed the decline of the heart rate and blood pressure caused by serotonin or 2-methylserotonin dose-dependently.

The depressing action of a compound of the present invention against Bezold-Jarish (BJ) reflex caused by serotonin observed in a rat is shown in the table below.

| Example No. | Depressing action against BJ reflex (ED₅₀; µg/kg i.v.) |
|---|---|
| 5 | 0.048 |

### 2) Depressing action against vomit caused by carcinostatic agents

Hypodermic or oral administration of the compound of the present invention in an amount of 0.01 to 1 mg/kg to a male ferret weighing from 1 to 1.5 kg depressed the vomit caused by intraperitoneal administration of 10 mg/kg cisplatin.

### 3) Depressing action against stress defecation

A nine-week-old male Wister rat was put in a cage for constraint stress, and the number of feces excreted was measured. A compound of the present invention or a salt thereof, when intravenously administered (1 to 100 µg/kg), depressed acceleration of defecation caused by constraint stress dose-dependently.

In addition, the compounds of this invention are low in toxicity, and the acute toxicity value with male mice was 25 to 50 mg/kg i.v. (up and down method).

Pharmaceutical compositions containing at least one kind of the compounds of the present invention or salts thereof as an active ingredient are made by the use of a carrier, an excipient and other commonly employed additives in the form of tablets, powders, fine granules, capsules, pills, solutions, injections, suppositories, ointments and adhesive plasters, which are administered orally (inclusive of sublingual administration) or parenterally.

As the carrier and excipient for the manufacture of these pharmaceutical compositions, may be used solid or liquid non-toxic substances for medicinal use, such as lactose, magnesium stearate, starch, talc, gelatin, agar, pectin, gum arabic, olive oil, sesame oil, cacao butter, ethylene glycol and other compounds commonly employed.

The clinical dosage of the compounds of the present invention is appropriately determined by taking, into consideration, the illness conditions, body weight, age, sex distinction and other factors of the patient being treated, but is generally 0.1 to 1.0 mg/day for intravenous injection and 0.5 to 50 mg/day for oral administration to adults (in a single or several divided doses).

### (Examples)

The following Reference Example, Examples and Example of Formulation will further illustrate the invention in detail.

### Reference Example

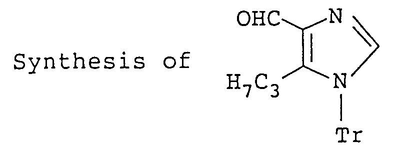

(1) To 8 ml of anhydrous tetrahydrofuran, was added 0.29 g of 60% oily solution of sodium hydride under an argon atmosphere, 1.09 g of ethyl 5-n-propyl-4-imidazolylcarboxylate was then added at a temperature of 5°C or lower for a period of ten minutes, and the resulting mixture was stirred at room temperature for one hour. After cooling again to a temperature of 5°C or lower, 1.67 g of chlorotriphenylmethane was added for a period of ten minutes, the mixture was stirred overnight at room temperature, water was then added, and the pH was adjusted to 8 by addition of hydrochloric acid. The reaction mixture was extracted with chloroform, the extract was dried over anhydrous magnesium sulfate, the solvents were distilled off from the dried solution under reduced pressure, and the residue was treated by column chromatography on silica gel (eluent: a mixture of chloroform and methanol), thus giving 0.65 g of ethyl 5-n-propyl-1-triphenylmethyl-4-imidazolylcarboxylate. Physicochemical properties:
   (i) Mass spectrum (EI), m/z: 424 (M⁺)
   (ii) NMR (CDCl₃)
      - δ:: 0.2-0.5 (m, 5H), 1.4 (t, 3H), 2.3-2.5 (m, 2H), 4.35 (q, 2H), 7.0-7.4 (m, 16H)
(2) To a solution of the above compound (0.65 g) in 7 ml dichloromethane, was added dropwise 1.02M toluene solution of diisobutylaluminum hydride (4.25 ml) at a temperature of 5°C or lower for a period of ten minutes under an argon atmosphere, and the mixture was stirred for three hours at a temperature of 5°C or lower and then for three hours at room temperature. A saturated aqueous solution of sodium sulfate was added to the reaction mixture, the aluminum complex thus formed was filtered off by using Celite, the filtrate was concentrated, 15 ml dioxane was added and 1 g manganese dioxide was added to the filtrate, the mixture was stirred at 90°C for eight hours, and the insoluble matters were filtered off by using Celite. The solvents were distilled off from the filtrate under reduced pressure, a mixture of diethyl ether and diisopropyl ether was added to the residue, and the crystals thus formed were collected by filtration, thus giving 0.19 g of 5-n-propyl-1-triphenylmethyl-4-imidazolylcarboxyaldehyde.
   Physicochemical properties:
   (i) Mass spectrum (EI), m/z: 380 (M⁺)
   (ii) NMR (CDCl₃)
      - δ:: 0.25-0.6 (m, 5H), 2.2-2.55 (m, 2H), 7.0-7.5 (m, 16H), 10.0 (S, 1H)

### Example 1

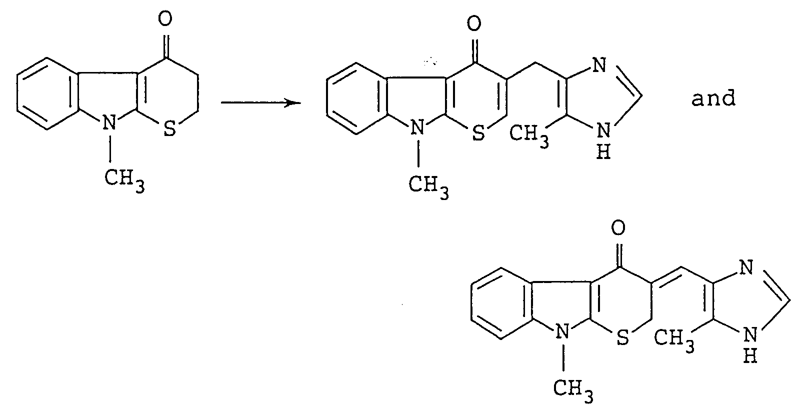

To a solution of 2.77 g 9-methyl-2,3,4,9-tetrahydrothiopyrano[2,3-b]indol-4-one in 100 ml anhydrous tetrahydrofuran, was added a solution of lithium isopropylamide (prepared by adding 5.8 ml of 2.5M hexane solution of n-butyl lithium to a solution of 1.97 ml diisopropylamine in anhydrous tetrahydrofuran at -78°C, slowly raising the temperature to 0°C, and again cooling to -78°C) at -78°C for a period of 20 minutes under an argon atmosphere, the mixture was stirred for 30 minutes at this temperature and then for 30 minutes at 0°C. The mixture was cooled to -78°C again, and 4.5 g of 5-methyl-1-(triphenylmethyl)imidazole-4-carboxyaldehyde was then added for a period of 20 minutes. The temperature of the resulting solution was slowly raised to 5°C for a period of two hours, and stirred overnight at this temperature. After cooling again to -78°C, 2 ml acetic acid and 19.4 g p-toluenesulfonic acid were added in that order, the temperature of the resulting mixture was raised to room temperature and then heated under reflux for four hours, and the solvent was distilled off under reduced pressure. The residue was weakly alkalized by adding a saturated aqueous solution of sodium bicarbonate, and this solution was extracted with dichloromethane. The organic layer was dried, the solvent was distilled off from the dried solution under reduced pressure, and the residue was treated by column chromatography on silica gel (eluent: a mixture of chloroform and methanol), thus giving 0.71 g of 9-methyl-3-[(5-methyl-4-imidazolyl)methylene]-2,3,4,9-tetrahydrothiopyrano[2,3-b]indol-4-one [R_{f} value of 0.5 when subjected to thin-layer chromatography using a product of Merck Co. (eluent: a 6:1 mixture of chloroform and methanol)] and 0.05 g of 4,9-dihydro-9-methyl-3-[(5-methyl-4-imidazolyl)methyl]thiopyrano[2,3-b]-indol-4-one (R_{f} value: 0.65).
(1) 4,9-Dihydro-9-methyl-3-[(5-methyl-4-imidazolyl)methyl]thiopyrano[2,3-b]indol-4-one
Physicochemical properties:
(i) Melting point: 217-219°C (chloroform-isopropyl ether)
(ii) Elemental analysis (for C₁₇H₁₅N₃OS·0.11H₂O)

| | C(%) | H(%) | N(%) | S(%) |
|---|---|---|---|---|
| Calcd.: | 65.57 | 4.93 | 13.50 | 10.30 |
| Observed: | 65.56 | 4.82 | 13.36 | 10.59 |

(iii) Mass spectrum (EL), m/z: 309 (M⁺)
(2) 9-Methyl-3-((5-methyl-4-imidazolyl)methylene]2,3,4,9-tetrahydrothiopyrano[2,3-b]indol-4-one
Physicochemical properties:
(i) Melting point: 195-202°C (dec.) (chloroform-ethyl acetate)
(ii) Elemental analysis (for C₁₇H₁₅N₃OS·0.12CH₃COOC₂H₅)

| | C(%) | H(%) | N(%) | S(%) |
|---|---|---|---|---|
| Calcd.: | 65.62 | 5.03 | 13.13 | 10.02 |
| Observed: | 65.69 | 5.00 | 12.84 | 10.28 |

(iii) Mass spectrum (EI), m/z: 309 (M⁺)

### Example 2

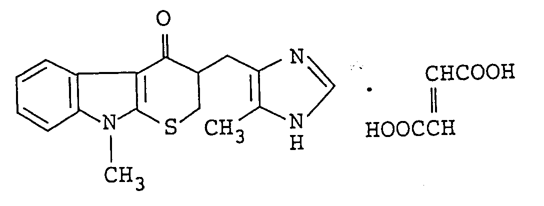

9-Methyl-3-[(5-methyl-4-imidazolyl)methylene]-2,3,4,9-tetrahydrothiopyrano[2,3-b]indol-4-one (0.65 g) was added to a mixture of 40 ml methanol and 1 ml 4N-HCl solution in dioxane, 1 g of 5% palladium-carbon was then added, and hydrogenation was carried out for one hour under ordinary pressure. The insoluble matters were filtered off from the reaction mixture, the solvents were distilled off from the filtrate, the residue was weakly alkalized by adding a saturated aqueous solution of sodium bicarbonate, and this solution was extracted with chloroform. The extract was dried, the solvent was distilled off under reduced pressure, and the residue was treated by column chromatography on silica gel (eluent: a mixture of chloroform and methanol), and the eluted product was treated with fumaric acid and recrystallized from a mixture of methanol and acetonitrile, thus giving 0.18 g of 9-methyl-2,3,4,9-tetrahydro-3-[(5-methyl-4-imidazolyl)methyl]-thiopyrano[2,3-b]indol-4-one fumarate.
Physicochemical properties:
(i) Melting point: 193-195°C
(ii) Elemental analysis (for C₁₇H₁₇N₃OS·C₄H₄O₄·0.3H₂O)

| | C(%) | H(%) | N(%) | S(%) |
|---|---|---|---|---|
| Calcd.: | 58.27 | 5.03 | 9.71 | 7.41 |
| Observed: | 58.12 | 4.93 | 9.93 | 7.35 |

(iii) Mass spectrum (EI), m/z: 311 (M⁺)

### Example 3

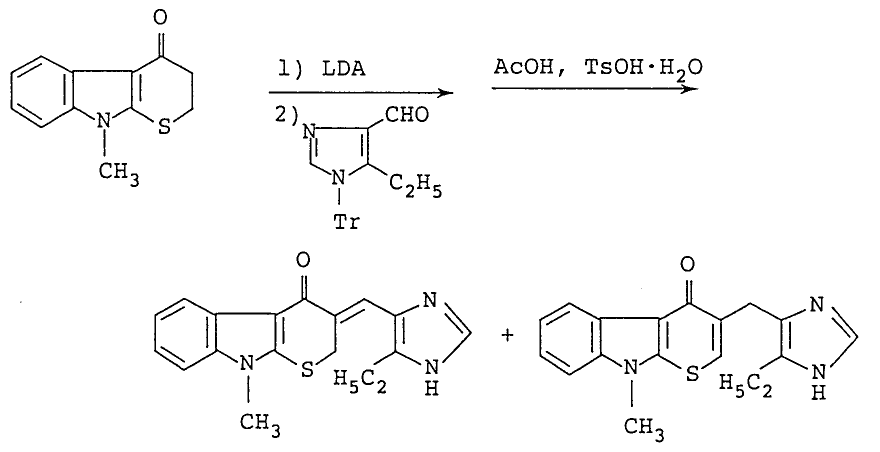

To a solution of 0.82 g 9-methyl-2,3,4,9-tetrahydrothiopyrano[2,3-b]indol-4-one in 30 ml tetrahydrofuran, was added dropwise a tetrahydrofuran solution containing 1.1 molar equivalent of lithium diisopropylamide (LDA) at -78°C. The mixture was stirred at -78°C for 30 minutes, then heated to -40°C and again cooled to -78°C. To the reaction mixture, was added 1.4 g 5-ethyl-1-triphenylmethyl-4-imidazolecarboxyaldehyde, the mixture was stirred overnight at 0°C, again cooled to -78°C, and 0.59 ml acetic acid was added. The temperature of the mixture was raised to room temperature, 5.8 g toluenesulfonic acid hydrate was added, and the resulting mixture was heated under reflux for four hours. The solvent was distilled off from the reaction mixture under reduced pressure, the residue was washed with diethyl ether, an aqueous solution of sodium carbonate was then added, and the resulting solution was extracted with chloroform. The extract was dried over anhydrous magnesium sulfate, the solvent was distilled off from the dried solution, and the residue was purified by column chromatography on silica gel, thus giving 0.3 g of 3-[(5-ethyl-4-imidazolyl)methylene]-9-methyl-2,3,4,9-tetrahydrothiopyrano[2,3-b]indol-4-one and 3-[(5-ethyl-4-imidazolyl)methyl]-9-methyl-4,9-dihydrothiopyrano[2,3-b]indol-4-one.
(1) 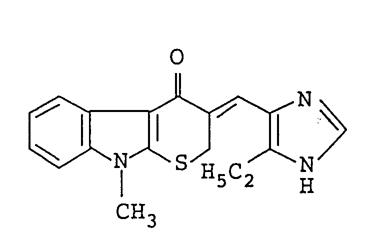
3-[(5-Ethyl-4-imidazolyl)methylene]-9-methyl-2,3,4,9-tetrahydrothiopyrano[2,3-b]indol-4-one
Physicochemical properties:
(i) Melting point: 190-195°C
(ii) Elemental analysis (for C₁₈H₁₇N₃OS·0.1H₂O·0.1AcOEt)

| | C(%) | H(%) | N(%) | S(%) |
|---|---|---|---|---|
| Calcd.: | 66.16 | 5.43 | 12.58 | 9.60 |
| Observed: | 66.16 | 5.36 | 12.33 | 9.85 |

(iii) Mass spectrum (FAB), m/z: 323 (M⁺+1)
(2) 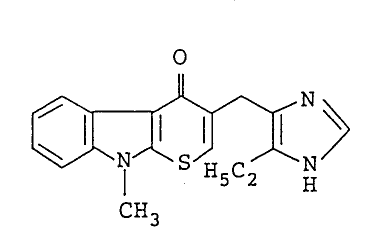
3-[(5-Ethyl-4-imidazolyl)methyl]-9-methyl-4,9-dihydrothiopyrano[2,3-b]indol-4-one (oily compound)
Physicochemical properties:
(i) NMR (CDCl₃)
   - δ:: 1.28 (3H, t), 2.74 (2H, z), 3.68 (3H, s), 4.12 (2H, S), 6.87 (1H, s), 7.2-7.4 (3H, m), 7.60 (1H, s), 8.3-8.4 (1H, m)

(ii) Mass spectrum (FAB), m/z: 323 (M⁺+1)

The following compounds were obtained in the similar way as in Example 3.

### Example 4

(1) 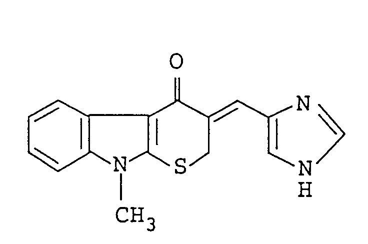
3-[(4-Imidazolyl)methylene]-9-methyl-2,3,4,9-tetrahydrothiopyrano[2,3-b]indol-4-one Physicochemical properties:
(i) Melting point: 218-221°C
(ii) Elemental analysis (for C₁₆H₁₃N₃OS)

| | C(%) | H(%) | N(%) | S(%) |
|---|---|---|---|---|
| Calcd.: | 65.06 | 4.44 | 14.23 | 10.86 |
| Observed: | 64.76 | 4.50 | 14.06 | 11.03 |

(iii) Mass spectrum (FAB), m/z: 296 (M⁺+1)
(2) 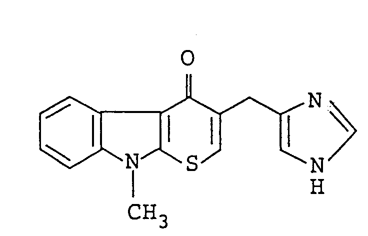
3-[(4-Imidazolyl)methyl]-9-methyl-4,9-dihydrothiopyrano[2,3-b]indol-4-one
Physicochemical properties:
(i) Melting point: 184-190°C
(ii) Mass spectrum (FAB), m/z: 296 (M⁺+1)
(iii) NMR (CDCl₃+CD₃OD)
   - δ:: 3.68 (3H, s), 4.12 (2H, s), 6.88 (1H, s), 7.2-7.4 (4H, m), 7.68 (1H, s), 8.3-8.4 (1H, m)

### Example 5

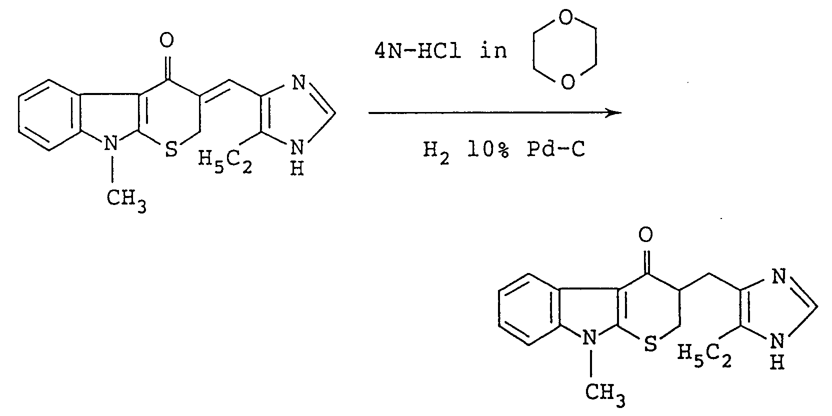

To a solution of 0.3 g 3-[(5-ethyl-4-imidazolyl)methylene]-9-methyl-2,3,4,9-tetrahydrothiopyrano[2,3-b]indol-4-one in a mixture of 30 ml methanol and 440 ml 4N-HCl in dioxane, was added 440 mg of 10% palladium-carbon, and hydrogenation was carried out at room temperature. After the reaction, the catalyst was filtered off and washed with hot methanol, the washings were put together with the filtrate, and the solvents were distilled off from combined solution under reduced pressure. An aqueous solution of sodium carbonate was added to the residue, the mixture was extracted with dichloromethane, and the extract was dried over anhydrous magnesium sulfate, and filtered. The solvent was distilled off from the filtrate under reduced pressure, and the residue was purified by chromatography on silica gel, thus giving 100 mg of 3-[(5-ethyl-4-imidazolyl)methyl]-9-methyl-2,3,4,9-tetrahydrothiopyrano[2,3-b]indol-4-one.
Physicochemical properties:
(i) Melting point: 233-237°C
(ii) Elemental analysis (for C₁₈H₁₉N₃OS)

| | C (%) | H (%) | N (%) | S (%) |
|---|---|---|---|---|
| Calcd.: | 66.07 | 5.91 | 12.84 | 9.80 |
| Observed: | 65.82 | 5.97 | 12.64 | 9.74 |

(iii) Mass spectrum (FAB), m/z: 325 (M⁺+1)

The following compound was obtained in the similar way as in Example 5.

### Example 6

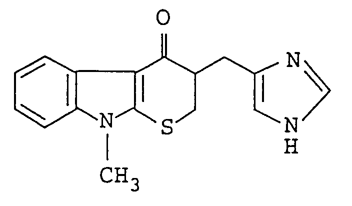

3-[(4-Imidazolyl)methyl]-9-methyl-2,3,4,9-tetrahydrothiopyrano[2,3-b]indol-4-one
Physicochemical properties:
(i) Melting point: 75-79°C
(ii) Elemental analysis (for C₁₆H₁₅N₃OS·0.6H₂O·0.5AcOEt)

| | C (%) | H (%) | N (%) | S (%) |
|---|---|---|---|---|
| Calcd.: | 61.38 | 5.78 | 11.93 | 9.10 |
| Observed: | 61.27 | 5.45 | 12.15 | 8.81 |

(iii) Mass spectrum (FAB), m/z: 298 (M⁺+1)

### Example 7

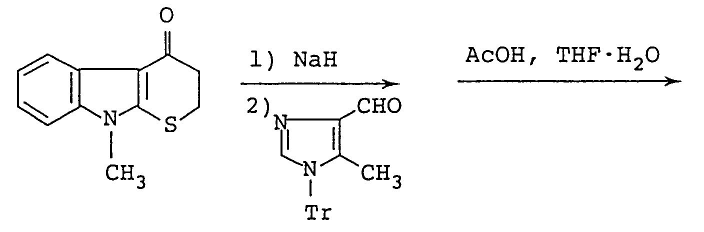

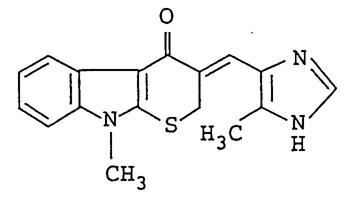

To a mixture of 10.0 g 9-methyl-2,3,4,9-tetrahydrothiopyrano[2,3-b]indol-4-one, 26.3 g 5-methyl-1-triphenylmethyl-4-imidazolylcarboxyaldehyde (containing one mole of dioxane), 30 g molecular sieves (3A) and 200 ml toluene, was added 0.37 g of 60% oily solution of sodium hydride under an argon atmosphere, and the resulting mixture was stirred at 100°C for three hours. After cooling the reaction mixture, 1.5 ml acetic acid was added, the insoluble matters were filtered off by the use of Celite, and the solvents were distilled off from the filtrate under reduced pressure. A mixture of water, acetic acid and tetrahydrofuran (each 100 ml) was added to the residue, the mixture was refluxed for 1.5 hours with stirring, the solvents were distilled off under reduced pressure, 300 ml toluene was then added, and the resulting mixture was extracted twice with 150 ml of 1N-HCl. The extract was neutralized with potassium carbonate and then extracted twice with chloroform (each 150 ml), this extract was dried over anhydrous magnesium sulfate, the solvent was distilled off from the dried solution, and the residue was washed with ethyl acetate, thus giving 8.78 g of 9-methyl-3-[(5-ethyl-4-imidazolyl)methylene]-2,3,4,9-tetrahydrothiopyrano[2,3-b]indol-4-one (identified as the same compound as that obtained in Example 1-(2)).

### Example 8

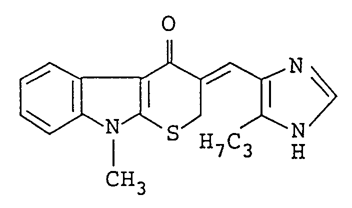

9-Methyl-3-[(5-n-propyl-4-imidazolyl)methylene]-2,3,4,9-tetrahydrothiopyrano[2,3-b]indol-4-one was obtained in much the same way as in Example 7, except that 5-n-propyl-1-triphenylmethyl-4-imidazolyl-carboxyaldehyde was employed in place of 5-methyl-1-triphenylmethyl-4-imidazolyl-carboxyaldehyde. Physicochemical properties:
(i) Mass spectrum (EI), m/z: 337 (M⁺), 304 (M⁺-SH)

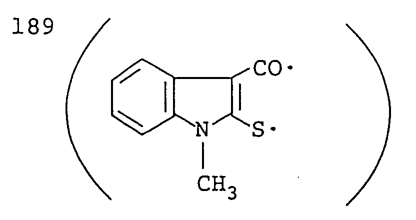

### Example 9

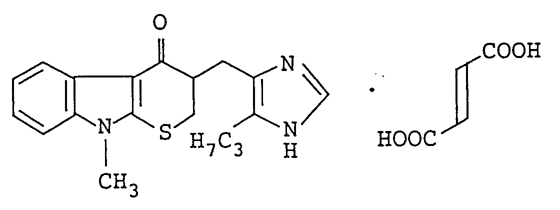

9-Methyl-3-[(5-n-propyl-4-imidazolyl)methyl]-2,3,4,9-tetrahydrothiopyrano[2,3-b]indol-4-one fumarate was obtained by hydrogenation of the compound of Example 8 in the similar way as in Example 2.
Physicochemical properties:
(i) Mass spectrum (EI), m/z: 399 (M⁺),

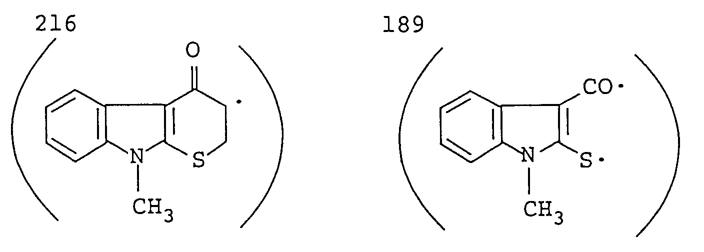

### Example of Formulation

Shown below are the examples of formulations using the compounds of the present invention as drugs.

### (1) Tablets

| | |
|---|---|
| Compound of Example 5 (hereinafter expressed as Compound A) | 0.2 mg |
| Lactose | 106.4 mg |
| Corn starch | 48.0 mg |
| Hydroxypropylcellulose | 4.8 mg |
| Magnesium stearate | 0.6 mg |
| | 160.6 mg/tablet |

To a uniform mixture of 200 mg Compound A, 106.4 g lactose and 48 g corn starch, is added 48 ml of 10% aqueous solution of hydroxypropylcellulose, and granulation is carried out by the use of a granulator. Magnesium stearate (0.6 g) is added to the obtained granules, and 1000 tablets each weighing 160 mg are made.

### (2) Powders

| | |
|---|---|
| Compound A | 0.4 mg |
| Mannitol | 770.0 mg |
| Corn starch | 199.6 mg |
| Polyvinyl pyrrolidone | 30.0 mg |
| | 1000.0 mg |

To a uniform mixture of 0.4 g Compound A, 770 g mannitol and 199.6 g corn starch, is added 300 ml of a 10% aqueous solution of polyvinyl pyrrolidone, and granulation is carried out by the use of a granulator to form powders (1 kg).

### (3) Capsules

| | |
|---|---|
| Compound A | 0.2 mg |
| Corn starch | 198.8 mg |
| Calcium stearate | 1.0 mg |
| | 200.0 mg |

A uniform mixture of 0.2 mg Compound A, 198.8 mg corn starch and 1 g calcium stearate is capsulated in No. 3 capsules (200 mg in each), thus forming 1000 capsules.

## Claims

1. A thiopyrano[2,3-b]indole derivative represented by the general formula: 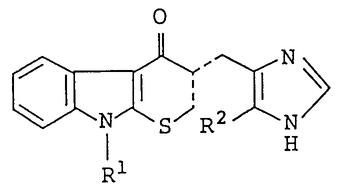 (wherein R¹ and R² are the same or different from each other and each represents a hydrogen atom or a lower alkyl group; and the broken lines mean that both are single bonds, or either one of them is a double bond) or a pharmacologically acceptable salt thereof.

2. The compound as described in Claim 1, wherein R¹ in the general formula in claim 1 is a methyl group and both of the broken lines are single bonds.

3. The compound as described in Claim 1 or 2, wherein the compound represented by the general formula in Claim 1 is 3-[(5-ethyl-4-imidazolyl)methyl]-9-methyl-2,3,4,9-tetrahydrothiopyrano[2,3-b]indol-4-one or a salt thereof.

4. A pharmaceutical composition comprising a thiopyrano[2,3-b]indole derivative represented by the general formula: 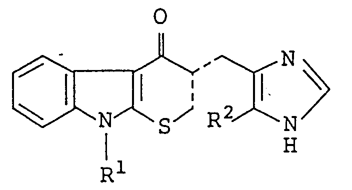 (wherein R¹ and R² are the same or different from each other and each represents a hydrogen atom or a lower alkyl group; and the broken lines mean that both are single bonds, or either one of them is a double bond) or a pharmacologically acceptable salt thereof, and a pharmacologically acceptable carrier.

5. The pharmaceutical composition as described in Claim 4, wherein R¹ in the general formula in Claim 4 is a methyl group and both of the broken lines are single bonds.

6. The pharmaceutical composition as described in Claim 4 or 5, wherein the compound represented by the general formula in Claim 4 is 3-[(5-ethyl-4-imidazolyl)methyl]-9-methyl-2,3,4,9-tetrahydrothiopyrano[2,3-b]indol-4-one or a salt thereof.

7. A method of antagonizing the physiological activity of 5-HT₃ in living bodies comprising administering an effective amount of a pharmaceutical composition as in Claim 4.

8. A method of antagonizing the physiological activity of 5-HT₃ in living bodies comprising administering an effective amount of a pharmaceutical composition as in Claim 5.

9. The method as in Claim 7, wherein said compound is 3-[(5-ethyl-4-imidazolyl)methyl]-9-methyl-2,3,4,9-tetrahydrothiopyrano[2,3-b]indol-4-one or a salt thereof.

10. A process for producing a thiopyrano[2,3-b]indole derivative represented by the general formula (I): 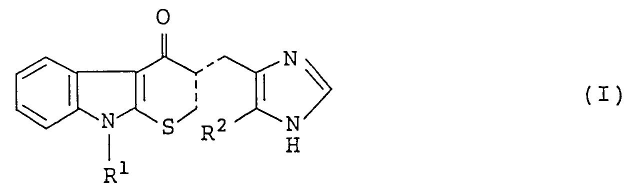 (wherein R¹ and R² are the same as below; and the broken lines mean that both are single bonds, or either one of them is a double bond), which comprises reacting a tetrahydrothiopyrano[2,3-b]indole compound represented by the general formula (II): 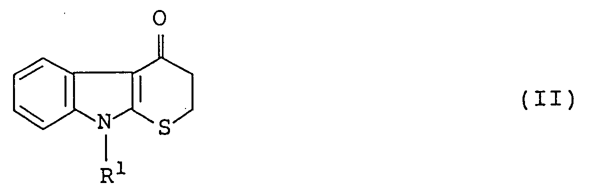 (wherein R¹ is a hydrogen atom or a lower alkyl group) with an imidazole derivative represented by the general formula (III): 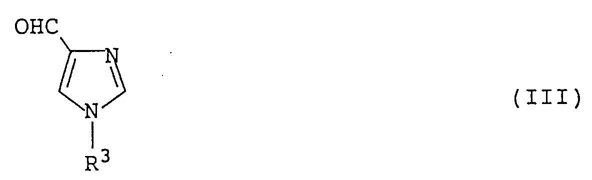 (wherein R³ is an arylmethyl group) to form a compound represented by the general formula (Ia): 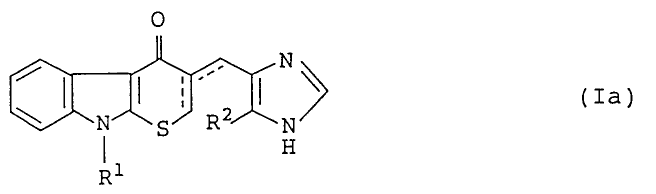 (wherein R¹ is the same as above; R² is a hydrogen atom or a lower alkyl group; and the lines ----- mean that either one of them is a double bond), and optionally reducing the compound (Ia) to form a compound represented by the general formula (Ib): 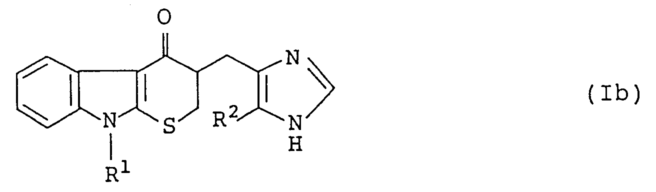 (wherein R¹ and R² are the same as above).

11. The process as described in Claim 10, wherein R¹ in the general formulas in Claim 10 is a methyl group and any of the broken lines is a single bond.

12. The process as described in Claim 10 or 11, wherein R¹ in the general formulas in Claim 10 is a methyl group, R² is an ethyl group, and any of the broken lines is a single bond.

13. A process for producing a compound represented by the general formula (Ib): 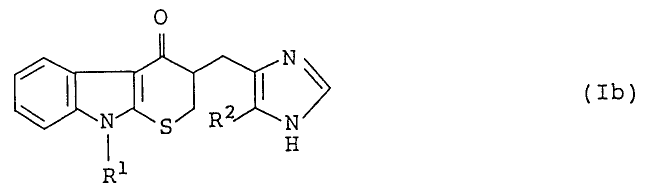 (wherein R¹ and R² are the same as below) comprising oxidizing a compound represented by the general formula (IV): 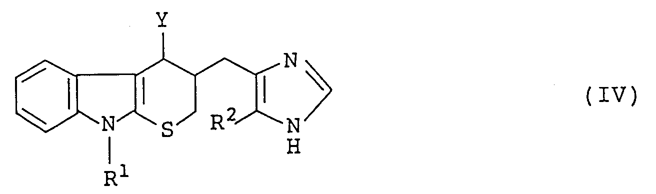 (wherein R¹ and R² are the same or different from each other and each represents a hydrogen atom or a lower alkyl group; and Y is a hydrogen atom or a hydroxyl group) by the use of an oxidizing agent.

14. The process as described in Claim 13, wherein R¹ in the general formulas in Claim 13 is a methyl group.

15. The process as described in Claim 13 or 14, wherein R¹ in the general formulas in Claim 13 is a methyl group and R² is an ethyl group.
